(19)

**Europäisches Patentamt**
**European Patent Office**
**Office européen des brevets**

(11) **EP 1 415 139 B1**

(12) **EUROPEAN PATENT SPECIFICATION**

(45) Date of publication and mention
of the grant of the patent:
**12.06.2013 Bulletin 2013/24**

(21) Application number: **02738307.4**

(22) Date of filing: **17.05.2002**

(51) Int Cl.:
*G01N 5/02* *(2006.01)*      *G01N 33/543* *(2006.01)*

(86) International application number:
**PCT/GB2002/002222**

(87) International publication number:
**WO 2002/095365 (28.11.2002 Gazette 2002/48)**

(54) **METHOD FOR DETERMINING A MASS CHANGING EVENT**

VERFAHREN ZUR BESTIMMUNG EINES DIE MASSE ÄNDERNDEN EREIGNISSES

PROCEDE POUR DETERMINER UN CHANGEMENT DE MASSE

(84) Designated Contracting States:
**AT BE CH CY DE DK ES FI FR GB GR IE IT LI LU MC NL PT SE TR**

(30) Priority: **18.05.2001  GB 0112079**
**14.03.2002  GB 0206010**
**27.03.2002  GB 0207167**

(43) Date of publication of application:
**06.05.2004   Bulletin 2004/19**

(73) Proprietor: **Farfield Group Limited**
**Crewe**
**Cheshire CW1 6GU (GB)**

(72) Inventors:
• **FREEMAN, Neville, John**
**Utkinton,**
**Tarporley CW6 0XA (GB)**
• **RONAN, Gerard, A.,**
**c/o Farfield Sensors Limited**
**Leslie Hough Way,**
**Salford M6 6AJ (GB)**

• **SWANN, Marcus,**
**c/o Department of Physics**
**Durham DH1 3LE (GB)**

(74) Representative: **Stuttard, Garry Philip et al**
**Urquhart-Dykes & Lord LLP**
**Tower North Central**
**Merrion Way**
**Leeds LS2 8PA (GB)**

(56) References cited:
**WO-A-98/22807      US-A- 5 065 030**
**US-A- 5 120 131      US-A- 5 501 986**
**US-A- 6 127 183**

• **LU J R; ET AL: "Neutron Reflection from Wet Interfaces", JOURNAL OF THE CHEMICAL SOCIETY. FARADAY TRANSACTIONS, ROYAL SOCIETY OF CHEMISTRY, vol. 94, no. 8, 21 April 1998 (1998-04-21) , pages 995-1018, XP000767848, CAMBRIDGE, GB**

**Description**

The present invention relates to a method for determining a mass changing event in a material of interest (eg a chemical or biological material of interest).

Mass changes may occur when two or more molecules associate or dissociate in a specific interaction (eg in a protein binding system or DNA hybridisation). However associative or dissociative interactions of a non-specific nature (eg resulting from weak chemical forces such as London forces and dispersion phenomena) also lead to a mass change and as a consequence manifest themselves as undesirable background noise. This complicates interpretation of the measurement and it may be very difficult to determine the extent of the interaction between the two molecules and their subsequent behaviour. The extent of the interaction is (for example) of particular importance in the analysis of DNA fragments where the differences in the nucleotide sequences are frequently very small. Moreover, determining the molecular behaviour of a molecule such as a protein is crucial to the overall understanding of biological processes.

In biological fluids, there may be numerous proteins whose nature and behaviour are unknown. A number of such proteins may interact indiscriminately with a biosensor in events often described as "non-specific binding events". These non-specific binding events contribute to a signal which cannot be differentiated from the signal which results from the desired interaction ("the specific binding event"). Such non-specific binding events hamper biotechnologists and limit the utility of biochemical systems based on detector/sensor platforms by reducing the degree of confidence in the measurement (and in some circumstances rendering any measurement impossible). As a result, users are forced to carry out rigorous 'clean up' procedures on biological fluids to remove unwanted proteins and other extraneous material before seeking to measure the specific binding event(s).

Many inorganic and organic materials exhibit specific behaviour as a consequence of exposure to physical stimuli (eg temperature, solvation or pH) or to chemical stimuli. These stimuli are frequently unrelated to the stimulus of interest. Even when such stimuli are related to the stimulus of interest the measured response may be difficult (if not impossible) to interpret. This leads at best to an inaccuracy in the measurement and at worst to complete breakdown of the measurement method.

Lu et al, J. Chem. Soc. Far Trans., 94, 8, 9951018 discloses the use of neutron reflection to probe structures at wet surfaces. US-A-5120131 discloses sample detection by integrated optical interference. US-A-6127183 discloses the measurement of refractive index of an optical sensor to determine surface changes. US-A-5501986 discloses a piezoelectric crystal based specific binding assay.

The present invention seeks to improve the determination of the mass characteristics of a material of interest by exploiting the sensitivity of certain sensor devices to mass changing events at the molecular level. More particularly, the present invention relates to a method for determining a mass changing event in the material of interest by measuring the temporal response of a sensor device to which the material of interest is exposed.

Thus viewed from one aspect the present invention provides a method for determining a mass changing event in a material of interest in a localised environment, said method comprising:

(A) providing a sensor device having a sensor component capable of exhibiting a measurable response to a change in the localised environment caused by the mass changing event in the material of interest therein;
(B) introducing the material of interest into the localised environment;
(C) inducing the mass changing event in the material of interest;
(D) generating an output from the sensor component over a temporal range, wherein step (D) comprises:

(D1) irradiating the sensor component with electromagnetic radiation in TE mode to produce a first pattern of interference fringes;
(D2) irradiating the sensor component with electromagnetic radiation in TM mode to produce a second pattern of interference fringes;

(E) measuring the response of a characteristic of the output over the temporal range, wherein step (E) comprises:

(E1) measuring movements in the first pattern of interference fringes;
(E2) measuring movements in the second pattern of interference fringes;
(E3) calculating the phase shift of the sensor component in TM mode from the movements in the first pattern of interference fringes
(E4) calculating the phase shift of the sensor component in TE mode from the movements in the second pattern of interference fringes;
(E5) calculating the phase shift of the sensor component in TM mode relative to the phase shift of the sensor component in TE mode; and

(F) relating the phase shift of the sensor component in TM mode relative to the phase shift of the sensor component in TE mode to the mass changing event.

The temporal response of the characteristic of the output of the sensor component depends crucially upon the type of mass changing event. It is the sensitivity of the sensor component leading to the measurable variation in the temporal response which enables different mass changing events to be differentiated.

The sensor component may be capable of exhibiting a measurable response in a parameter selected from effective refractive index, a dielectric constant, a viscoelastic property, a frequency of oscillation, a thermal absorption/desorption parameter, the permeability, the absorption of energy or of energetic particles (such as x-rays, gamma rays, β-rays, electrons, neutrons, ions, light, microwaves, acoustic waves) or the particle size. For example, the sensor device may be one or more of the following types: surface plasmon resonance sensor devices, resonant mirror sensor devices, acoustic sensor devices (such as quartz crystal and surface acoustic wave devices (by using frequency decay techniques for example)) or electrical sensor devices (capable of measuring impedance at (for example) RF or microwave frequencies). Preferably the parameter is the effective refractive index.

In a preferred embodiment, step (D) comprises:

(D) irradiating the sensor component with electromagnetic radiation to generate an output over a temporal range.

The mass changing event may be a chemical (ie non-physical) mass changing event.

When molecules interact (*eg* bond) in a specific manner there is in general an increase in molecular density whilst when molecules interact in a non-specific manner there is no significant change in molecular density. By measuring the temporal response, the present invention can distinguish such events. Thus in a preferred embodiment, the mass changing event is a specific molecular (or atomic) interaction. For example, the mass changing event is a specific associative or dissociative molecular interaction.

Without wishing to be bound by any theoretical considerations, it is noted that (in general) when two or more molecules interact (*eg* bond) in a chemically active manner, molecular density changes (typically increasing) with respect to the molecular density of the individual molecules. The factors effecting the molecular density after the bonding event (or binding event as it is often referred to) include the strength and nature of the bond(s) and any consequential change in conformation. If (on the other hand) two molecules interact in a chemically inactive manner, the combined molecular mass may increase but the molecular density stays largely unchanged because the overall volume also increases. By measuring the temporal response, the present invention makes it possible to differentiate the two types of interaction. Similarly, the breaking of a chemical bond will result in a molecular density change (typically decreasing) which can be differentiated from a chemically inactive change. By providing a unique signature of the type of interaction taking place, the invention will be of significant utility, in particular in the field of biosensing where the background measurements often severely limit the utility of the measurement.

In a preferred embodiment of the method of the invention, the mass changing event is a binding event (*eg* chemical, biochemical or biological binding event). The mass changing event may be a specific binding event or a non-specific binding event. Preferably the mass changing event is a specific binding event.

The binding event may be a bond making or bond breaking event. The binding event may be an associative event (*eg* formation of a molecular composition) or a dissociative event (*eg* decomposition of a molecular composition). The change in molecular density will be specific to the nature of the binding event and the invention can therefore be used to advantageously differentiate formation of a chemical bond or molecular composition of interest from formation of similar chemical bonds or molecular compositions. In this way, the invention provides a unique signature of the molecule or composition which has been formed by the binding event.

This embodiment is particularly advantageous when the material of interest is a complex molecule such as a complex biological molecule (*eg* a protein or DNA). The material of interest may be an antigen and the specific binding event may be formation of an antibody/antigen specific binding pair. By way of example, where an antibody is the material of interest bound to the sensor component (*eg* within either a sensing waveguide or a sensing layer), specific binding of an antigen gives rise to a significant change in molecular density. On the other hand, non-specific binding gives rise to a less significant change in molecular density. By allowing these effects to be distinguished, this embodiment of the present invention permits effective integration into biochemical systems.

In a preferred embodiment of the method of the invention, the mass changing event is a conformational change of the material of interest (eg chemical or biological material of interest). For example, the conformational change may be a molecular rearrangement (*eg* following a binding event or a change in the ambient environment).

This embodiment advantageously permits materials of interest (*eg* molecules) which are very similar in nature but have different conformations to be differentiated *eg* proteins such as prions or normal and mutant forms of α-synucleins. The material of interest may be a protein which undergoes a conformational change by a change in temperature, pH or by interaction with an additional species. By way of specific example (but not wishing to be bound by theory), hexakinase on the sensor component (*eg* within either a sensing waveguide or a sensing layer) undergoes a conformational change in the presence of glucose giving rise to a change in molecular density. The present invention is sensitive to the molecular density change so as to permit the change in conformation to be determined.

In a preferred embodiment of the method of the invention, the material of interest is a nucleotide and the mass changing event is nucleotide complementation, preferably effective nucleotide complementation (*eg* the interaction of DNA sequences in a complementation process or the determination of DNA amplification end points). By way of example (but not wishing to be bound by theory), the material of interest is a nucleotide strand on the sensor component (*eg* within either a sensing waveguide or a sensing layer) which gives rise to a change in molecular density when it undergoes effective nucleotide complementation. The present invention is sensitive to changes in density at a molecular level so as to permit effective nucleotide complementation to be determined. Thus the degree of effective nucleotide complementation may be determined.

The mass changing event may be a physical (*ie* non-chemical) mass changing event. The physical mass changing event may be aggregation.

In a preferred embodiment, step (C) comprises: imposing a condition such as to induce the mass changing event. The condition may be a chosen temperature, pressure, acidity, solvent or humidity.

Before or after step (B), the method may comprise:

(1) irradiating the sensor component with electromagnetic radiation to generate a first output;
(2) measuring a characteristic of the first output; and wherein steps (E) and (F) are:

(E) measuring the response of a characteristic of the output over the temporal range relative to the characteristic of the first output; and
(F) relating the response of the characteristic of the output over the temporal range relative to the characteristic of the first output to the mass changing event.

Steps (1) and (2) may be performed at start-up. The results may be stored electronically (*eg* as calibration data).

In a preferred embodiment, the sensor device is an interferometric sensor device. The sensor component of the interferometric sensor device may comprise at least one waveguide (eg a slab or channel waveguide) or a fibre optic component. For example, the sensor component may be a waveguide structure. The waveguide structure may be generally of the planar type disclosed in WO-A-98/22807 or WO-A-01/36945.

Preferably the sensor component is a waveguide structure including:

either (a) one or more sensing layers capable of inducing in a secondary waveguide a measurable response to a change in the localised environment caused by the mass changing event or (b) a sensing waveguide capable of exhibiting a measurable response to a change in the localised environment caused by the mass changing event.

In this embodiment, the mass changing event contributes to a change in the effective refractive index of the sensor component. The waveguide structure is particularly sensitive to changes in molecular density and this is advantageously exploited to differentiate mass changing events.

Particularly preferably the sensor component is a waveguide structure including:

either (a) one or more sensing layers capable of inducing in a secondary waveguide a measurable response to a change in the localised environment caused by the mass changing event and an inactive (eg deactivated) secondary waveguide in which the sensing layer is incapable of inducing a measurable response to a change in the localised environment caused by the mass changing event or (b) a sensing waveguide capable of exhibiting a measurable response to a change in the localised environment caused by the mass changing event and an inactive (eg deactivated) waveguide substantially incapable of exhibiting a measurable response to a change in the localised environment caused by the mass changing event.

Preferably each of the sensing waveguide or secondary waveguide (or any additional waveguides such as reference waveguides) of the sensor component is a planar waveguide (*ie* a waveguide which permits light propagation in any arbitrary direction within the plane). Particularly preferably each planar waveguide is a slab waveguide.

Preferably the sensor component constitutes a multi-layered structure (*eg* a laminated waveguide structure) of the types disclosed in WO-A-98/22807 and WO-A-01/36945 (Farfield Sensors Limited). In a preferred embodiment, each of the plurality of layers in the multi-layered sensor component are built onto a substrate (*eg* of silicon)-through known processes such as PECVD, LPCVD, etc. Intermediate transparent layers may be added (*eg* silicon dioxide) if desired. Typically the sensor component is a multilayered structure of thickness in the range 0.2-10 microns. A layered structure advantageously permits layers to be in close proximity (eg a sensing waveguide and an inactive (reference) waveguide may be in close proximity to one another so as to minimise the deleterious effects of temperature and other environmental factors). Preferably the sensor component comprises a stack of transparent dielectric layers wherein layers are placed in close proximity. Preferably each layer is fabricated to allow equal amounts of electromagnetic radiation to propagate by simultaneous excitation of the guided modes in the structure.

The characteristic of the output may be a positional characteristic. Preferably the output is a pattern of interference fringes which may be measured (step (E)) by a conventional measuring means (see for example WO-A-98/22807) eg one or more detectors such as photodetectors which measure the intensity of electromagnetic radiation. Preferably step (E) comprises: measuring movements in the pattern of interference fringes over the temporal range. Particularly preferably step (E) further comprises: calculating the phase shift from the movements in the pattern of interference fringes over the temporal range.

The characteristic of the output may be a non-positional characteristic. In a preferred embodiment, the non-positional characteristic of the pattern of interference fringes is the contrast (eg the difference in intensity between the outer fringe envelope and the inner fringe envelope). For example, the contrast may be the difference in intensity between the outer fringe envelope and the inner fringe envelope at a corresponding position in the pattern. Preferably the contrast may be the difference in intensity between the maxima of the outer fringe envelope and the maxima of the inner fringe envelope.

The measurement of non-positional characteristics of an interference pattern is discussed in co-pending UK patent application number 0207167.8 of Farfield Sensors Limited.

Preferably the sensor component is adapted so as to be usable in evanescent mode or whole waveguide mode.

Thus in a first embodiment, the sensor component includes one or more sensing layers capable of inducing in a secondary waveguide a measurable response to a change in the localised environment caused by the mass changing event. In this first embodiment, the sensor device is advantageously adapted to optimise the evanescent component so as to induce in the secondary waveguide a measurable response. The sensor component may comprise a plurality of separate sensing layers to enable mass changing events at different localised environments to be determined.

In a preferred embodiment, the sensing layer comprises an absorbent material (eg a polymeric material such as polymethylmethacrylate, polysiloxane, poly-4-vinylpyridine) or a bioactive material (eg containing antibodies, enzymes, DNA fragments, functional proteins or whole cells). The absorbent material may be capable of absorbing a gas, a liquid or a vapour containing a chemical material of interest. The bioactive material may be appropriate for liquid or gas phase biosensing. For example, the sensing layer may comprise a porous silicon material optionally biofunctionalised with antibodies, enzymes, DNA fragments, functional proteins or whole cells.

In a preferred method of the invention, the secondary waveguide comprises silicon oxynitride or silicon nitride.

In a second embodiment, the sensor component includes a sensing waveguide capable of exhibiting a measurable response to a change in the localised environment caused by the mass changing event. In this second embodiment, the sensor device is adapted to minimise the evanescent component and may be used advantageously in whole waveguide mode.

In a preferred embodiment, the sensing waveguide comprises an absorbent material (eg a polymeric material such as polymethylmethacrylate, polysiloxane, poly-4-vinylpyridine) or a bioactive material (eg containing antibodies, enzymes, DNA fragments, functional proteins or whole cells). The absorbent material may be capable of absorbing a gas, a liquid or a vapour containing a chemical material of interest. The bioactive material may be appropriate for liquid or gas phase biosensing. For example, the sensing waveguide may comprise a porous silicon material optionally biofunctionalised with antibodies, enzymes, DNA fragments, functional proteins or whole cells.

Where the sensor component comprises a sensing waveguide adapted for use in whole waveguide mode, an absorbent layer in the form of an overcoating may be present for use as a membrane (for example) to separate out certain stimuli.

To optimise the performance of the first embodiment, the sensor component may further comprise an inactive secondary waveguide in which the sensing layer is incapable of inducing a measurable response to a change in the localised environment caused by the mass changing event. The inactive secondary waveguide is capable of acting as a reference layer. It is preferred that the secondary waveguide and inactive secondary waveguide have identical properties with the exception of the response to the change in the localised environment caused by the mass changing event. By way of example, the secondary waveguide and inactive secondary waveguide are made of silicon oxynitride.

To optimise the performance of the second embodiment, the sensor component may further comprise an inactive (eg deactivated) waveguide substantially incapable of exhibiting a measurable response to a change in the localised environment caused by the mass changing event. The inactive waveguide is capable of acting as a reference layer. The physical, biological and chemical properties of the sensing waveguide and inactive waveguide are as similar as possible (with the exception of the response to the change in the localised environment caused by the mass changing event). Typically the inactive waveguide is made of silicon oxynitride.

As a consequence of mass changing events, changes in the dielectric properties (eg the effective refractive index) of the sensing waveguide or sensing layer occur. This causes a measurable response (ie a change in the transmission of electromagnetic radiation down the sensing waveguide (or waveguides) in whole waveguide mode or the secondary waveguide in evanescent field mode) which (in one embodiment) manifests itself as a movement of interference fringes. This differs according to whether the sensor component is interrogated in TE or TM mode.

By way of example, the movement of the pattern of interference fringes may be used to calculate the phase shift which takes place in the sensing waveguide or sensing layer during the passage of electromagnetic radiation through the sensor component. The phase shift is effectively directly proportional to changes occurring in the effective refractive index of the sensing waveguide or sensing layer and differs according to whether the sensor component is interrogated in TE or TM mode.

A pattern of interference fringes (*eg* for TE and TM modes respectively) may be generated when the electromagnetic radiation from the sensor component is coupled into free space and may be recorded in a conventional manner (see for example WO-A-98/22807). A response of the sensor component to a change in the localised environment may be measured from movement of the fringes in the interference pattern. The phase shift of the electromagnetic radiation in the sensor component (*eg* induced in the secondary waveguide in evanescent field mode or exhibited in the sensing waveguide in whole waveguide mode) may be calculated. In turn, the change in molecular density contributing to a change in the effective refractive index may be calculated.

Movement in the interference fringes may be measured either using a single detector which measures changes in the intensity of electromagnetic radiation or a plurality of such detectors which monitor the change occurring in a number-of fringes or the entire interference pattern. The one or more detectors may comprise one or more photodetectors (*eg* photodiodes). Where more than one photodetector is used this may be arranged in an array. In an array format, the relating means capable of relating the measurable response in TM mode and the measurable response in TE mode to a mass changing event may be deployed in a spatially resolved manner. Such spatial resolution can be achieved by means of (for example) remote imaging or lithography or scanning a measurement probe as in the case of an atomic microprobe.

In a preferred embodiment of the method of the invention, step (D) is carried out with electromagnetic radiation in TM mode.

In a preferred embodiment of the method of the invention, step (D) is carried out with electromagnetic radiation in TE mode.

In a preferred embodiment of the method of the invention, step (D) comprises:

(D1) irradiating the sensor component with electromagnetic radiation in TE mode to produce a first pattern of interference fringes;

(D2) irradiating the sensor component with electromagnetic radiation in TM mode to produce a second pattern of interference fringes;

and step (E) comprises:

(E1) measuring movements in the first pattern of interference fringes; and

(E2) measuring movements in the second pattern of interference fringes.

Particularly preferably step (E) of the method of the invention further comprises:

(E3) calculating the phase shift of the sensor component in TM mode from the movements in the first pattern of interference fringes;

(E4) calculating the phase shift of the sensor component in TE mode from the movements in the second pattern of interference fringes;

and step (F) is
relating the phase shift of the sensor component in TM mode and the phase shift of the sensor component in TE mode to the mass changing event.

More preferably step (E) of the method of the invention further comprises:

(E3) calculating the phase shift of the sensor component in TM mode from the movements in the first pattern of interference fringes

(E4) calculating the phase shift of the sensor component in TE mode from the movements in the second pattern of interference fringes;

(E5) calculating the phase shift of the sensor component in TM mode relative to the phase shift of the sensor component in TE mode;

and step (F) is

relating the the phase shift of the sensor component in TM mode relative to the phase shift of the sensor component in TE mode to the mass changing event.

Preferably the phase shift of the sensor component in TM mode relative to the phase shift of the sensor component in TE mode is a ratio of the phase shift of the sensor component in TM mode to the phase shift of the sensor component in TE mode.

Step (D) may comprise: generating an output from the sensor component on at least two occasions over a temporal range. Preferably step (D) comprises: generating an output from the sensor component continuously over a temporal range.

The mass changing event is typically determined qualitatively but may be determined quantitatively in terms of a change in molecular-density or mass.

Thus in a preferred embodiment, the method further comprises:

(G1) relating the response of the characteristic of the output over the temporal range to a change in the intrinsic refractive index and/or the volume; and
(G2) calculating the change in the molecular density; and
(G3) optionally calculating the change in mass.

Preferably the method further comprises:

(G1) relating the movements in the first pattern of interference fringes and second pattern of interference fringes to a change in the intrinsic refractive index and/or the volume; and
(G2) calculating the change in the molecular density; and (G3) optionally calculating the change in mass.

In a particularly preferred embodiment, step (G1) comprises:

(G1) relating the movements in the first pattern of interference fringes and second pattern of interference fringes to a change in the intrinsic refractive index and/or the thickness of the sensing layer or sensing waveguide.

Preferably the sensor device further comprises:

relating means capable of relating the measurable response to a change in molecular density in the localised environment.

The relating means enables the measurable response to be directly related to the molecular density or indirectly related to the molecular density (eg to the dimensionality such as the volume and to the mass) and may be for example a piezoelectric sensing system (where the measured resonant frequency is related to the mass and the decay time constant of free oscillation to the dimension) or an optical system such as a surface plasmon resonance device in combination with a device based on other analytical techniques such as ellipsometry.

Where volume is to be measured directly or indirectly, this may be by determination of one or more of the relevant dimensions or by displacement of the medium surrounding the volume.

In a preferred embodiment of the method, said sensor device further comprises:

first irradiating means for irradiating the sensor component with electromagnetic radiation in TM mode;
second irradiating means for irradiating the sensor component with electromagnetic radiation in TE mode;
measuring means for measuring the measurable response of the sensor component in TM mode and for measuring the measurable response of the sensor component in TE mode; and
relating means capable of relating the measurable response of the sensor component in TM mode and the measurable response of the sensor component in TE mode to a change in molecular density in the localised environment.

The first and second irradiating means may be the same or different. The measuring means may be one or more detectors in an array.

Preferably the sensor device further comprises:

a synchronising means for synchronising the measuring means with the first irradiating and second irradiating means so as to correlate the measurement of the measurable response of the sensor component in TE mode and of the measurable response of the sensor component in TM mode with the irradiation of the sensor component with electromagnetic radiation in TE and TM mode respectively.

Particularly preferably the synchronising means is capable of calculating the phase shift of the sensor component in TE mode and the phase shift of the sensor component in TM mode.

Particularly preferably the synchronising means is capable of relating the movements in the first pattern of interference fringes and second pattern of interference fringes to the mass changing event. -

The first and second irradiating means may be adapted to irradiate the sensor component with electromagnetic radiation in TE or TM mode sequentially or simultaneously. The first and second irradiating means may be the same or different. Where different sources of electromagnetic radiation are used, an optical switch (eg a rotating mirror) may be used to switch rapidly between the two. Alternatively, a single source of electromagnetic radiation may be used to simultaneously excite TE and TM modes of the sensor component by (for example) aligning the polarisation vector of the linearly polarised source at an angle with respect to the plane of the sensing waveguide or sensing layer of the sensor component. An active analyser system may be used to alternately remove the unwanted TE or TM mode radiation during data capture of the desired TM or TE output respectively. The active analyser system may comprise an electro-optic half wave plate placed in series with an analyser.

In a preferred embodiment, an adjustable analyser may be used to measure the first pattern of interference fringes and the second pattern of interference fringes separately. The measurements may be synchronised with the excitation and/or polarisation procedure to ensure that phase shift information is correlated with TE and TM excitation.

A controller may be provided to synchronise the one or more sources of electromagnetic radiation and one or more detectors. For example, the controller may capture the data from a photodetector (eg photodiode) array. The firing of the (or each) source of electromagnetic radiation may be synchronised by the controller with the alternate capture of the first and second pattern of interference fringes generated in TM mode and TE mode. The controller may be adapted to calculate the phase shift in TE and TM modes independently.

Electromagnetic radiation generated from a conventional source may be propagated into the sensor component in a number of ways. In a preferred embodiment, electromagnetic radiation is simply input via an end face of the sensor component (this is sometimes described as "an end firing procedure"). Preferably the electromagnetic radiation source provides incident electromagnetic radiation having a wavelength falling within the optical range. Propagating means may be employed for substantially simultaneously propagating incident electromagnetic radiation into a plurality of waveguides. For example, one or more coupling gratings or mirrors may be used. A tapered end coupler rather than a coupling grating or mirror may be used to propagate radiation into the lowermost waveguide. Preferably the amount of electromagnetic radiation in the sensing waveguide/inactive waveguide or in the secondary waveguide/inactive secondary waveguide is equal.

The incident electromagnetic radiation may be oriented (eg plane polarised) as desired using an appropriate polarising means. The incident electromagnetic radiation may be focussed if desired using a lens or similar micro-focussing means.

A plurality of electromagnetic radiation detector units (eg in an array) and/or a plurality of electromagnetic radiation sources may be used to measure in discrete areas of the sensor component simultaneously the responses to changes in the localised environment caused by the mass changing event. Alternatively, the position of the electromagnetic radiation detector and electromagnetic radiation source relative to the sensor component may be changed to provide information concerning responses in discrete areas of the sensor component. For example, discrete responses to a change in the localised environment caused by different mass changing events may be measured in discrete areas of the sensor component. For this purpose, the preferred assembly makes use of the versatility of the evanescent mode and comprises a plurality of separate sensing layers or regions.

The sensor component may be excited across its width and a two-dimensional photodiode array (or the like) may be used to effectively interrogate "strips" of the sensor component (eg an array sensor). This may be carried out across more than one axis simultaneously or sequentially to provide spatially resolved information relating to mass changing events on the sensor component.

Preferably the sensor device comprises:

means for intimately exposing at least a part of the (or each) sensing layer or the sensing waveguide of the sensor component to the localised environment.

In a preferred embodiment, the means for intimately exposing at least a part of the sensing layer or the sensing waveguide to the localised environment is integrated onto the sensor component.

Preferably the means for intimately exposing at least a part of the (or each) sensing layer or the sensing waveguide of the sensor component to the localised environment is as described in WO-A-01/36945. The means may be automated in order to reduce the requisite degree of user intervention.

Preferably the means for intimately exposing at least a part of the sensing layer or the sensing waveguide to the localised environment is adapted to permit the continuous introduction of an analyte containing a material of interest (ie a dynamic system). For example, it may permit the continuous introduction of the material of interest in a discontinuous flow (eg as a train of discrete portions) into the localised environment. This may be achieved by capillary action or by a separate urging means.

Preferably the means for intimately exposing at least a part of the sensing layer or the sensing waveguide to the localised environment is adapted to induce mass changing events (eg reactions) in a static analyte containing a chemical or biological material of interest. In this sense, the system may be considered to be dynamic. Mass changing events-(eg reactions) may be induced in any conventional manner such as by heat or radiation.

The means for intimately exposing at least a part of the (or each) sensing layer or the sensing waveguide to the localised environment may be a part of a microstructure positionable on the surface of and in intimate contact with the sensor component.

Preferably the microstructure comprises means for intimately exposing at least a part of the sensing layer or the sensing waveguide to the localised environment in the form of one or more microchannels and/or microchambers. For example, an analyte containing a chemical material of interest may be fed through microchannels or mass changing events (*eg* chemical reactions) may take place in an analyte containing a material of interest located in a microchamber. An analyte containing chemical material of interest may be fed into the microchannels by capillary action or positively fed by an urging means.

In a preferred embodiment, the means for intimately exposing at least a part of the (or each) sensing layer or the sensing waveguide to the localised environment is included in a cladding layer. For example, microchannels and/or microchambers may be etched into the cladding layer. The cladding layer may perform optical functions such as preventing significant discontinuities at the boundary of the sensing waveguide or sensing layer(s) or chemical functions such as restricting access of certain species to the sensing waveguide or sensing layer(s). The cladding layer may be integrated onto the sensor component.

Preferably the whole of or a portion of any additional functionality may be included in the cladding layer. In one embodiment, the sensing layer itself may be incorporated in the cladding layer (for example in the form of an absorbent material). Particularly preferably, the whole of the additional functionality may be provided in the cladding layer and include sensing devices such as for example quadrature electric field tracks or other microfluidic sensing devices. The cladding layer may incorporate an electromagnetic source (*eg* a laser) and/or means for detecting electromagnetic radiation (of the type detailed below). The cladding layer may incorporate a chemical separating means (eg an HPLC based device).

Preferably the means for exposing at least a part of the (or each) sensing layer or the sensing waveguide of the sensor component to the localised environment is a sensor cell. Preferably the sensor cell has a low volume (*eg* 50microlitres or less). By keeping the cell volume small, the method of the invention is not hampered and the amount of protein consumed is advantageously minimised.

The present invention will now be described in a non-limitative sense with reference to the Examples and accompanying Figures in which:

> Figure 1 represents schematically in plan view a sensor device for use in an embodiment of the invention;
> Figure 2 represents schematically in plan view a sensor device for use in an embodiment of the invention;
> Figure 3 represents schematically in plan view a sensor device for use in an embodiment of the invention;
> Figure 4 illustrates 20 oligonucleotide DNA hybridisation using a AnaLight Bio250 (Farfield Sensors Limited);
> Figure 5 illustrates a study of a metabolic pathway on the AnaLight Bio250;
> Figure 6 illustrates schematically the sensor component used in the Examples; and
> Figure 7 illustrates TE vs TM for determining a unique combination of intrinsic refractive index and thickness.

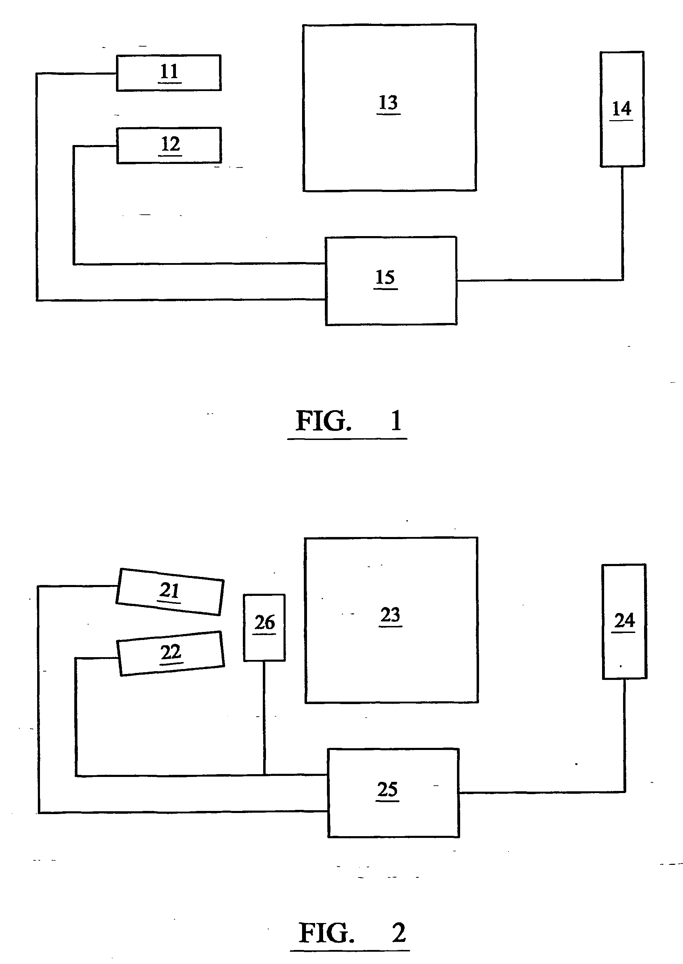

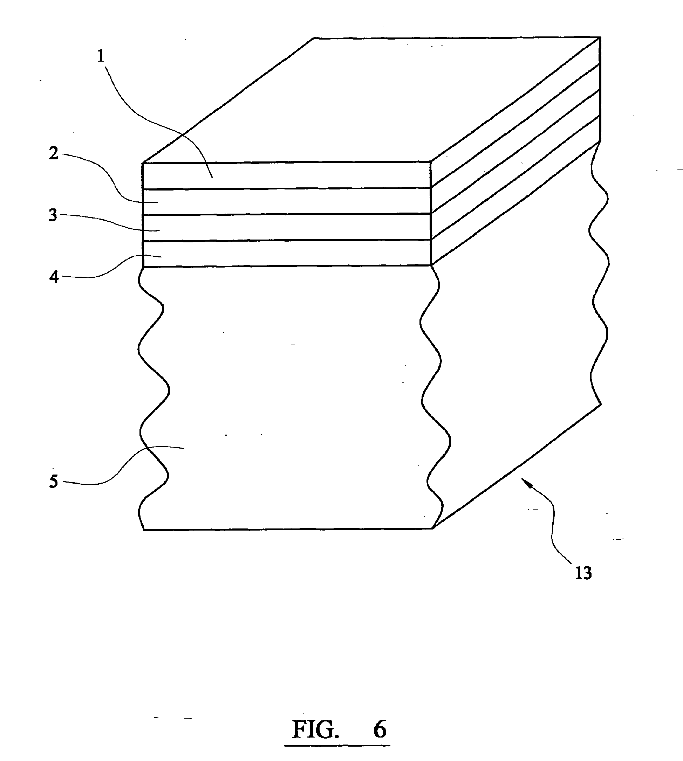

A sensor device for performing an embodiment of the method of the invention is shown schematically in Figure 1. Plane polarised electromagnetic radiation is generated in TM mode using a first electromagnetic radiation source (11) and in TE mode using a second electromagnetic radiation source (12) relative to the sensor component (13). The sensor component (13) is of a multi-layered type as described in WO-A-98/22807 and illustrated in Figure 6.

An interference pattern is captured by the photodiode or photodiode array (14). The two electromagnetic radiation sources (11 and 12) are controlled electronically by a controller (15) that also captures the data from the photodiode array (14). The firing of each of the electromagnetic radiation sources (11) and (12) is synchronised by the controller (15) such that the interference patterns in TM mode and TE mode are alternately captured. The controller (15) calculates the phase shift in each mode and the TE and TM phase shifts are compared in order to measure the change in molecular density. This data may be reported directly or made available to secondary systems for further analysis.

A further embodiment is shown schematically in figure 2 in which a rotating mirror (26) is employed to switch rapidly between electromagnetic radiation in TE and TM mode from first and second electromagnetic radiation sources (21) and (22) respectively for irradiating sensor component (13). A controller (25) is arranged to synchronise the electromagnetic radiation sources (21) and (22), the optical switch (26) and the photodiode array (24).

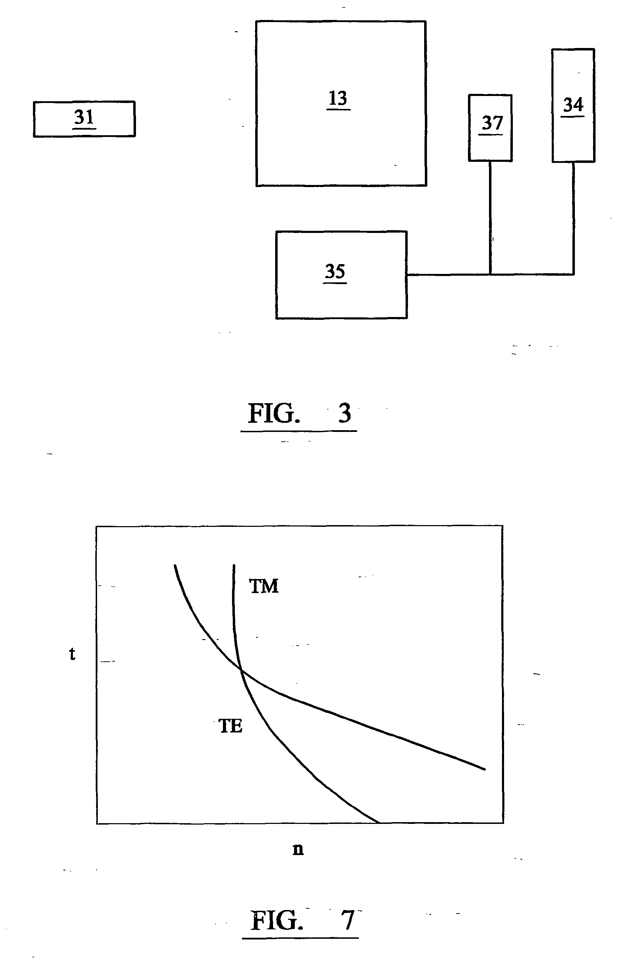

In figure 3, a single electromagnetic radiation source (31) is used to simultaneously irradiate the sensor component (13) with electromagnetic radiation in TE and TM modes. This is achieved by aligning the polarisation vector of the linearly polarised optical source at an angle with respect to the plane of the sensing layer or sensing waveguide of the sensor component (13). An active analyser system (37) is used to remove unwanted output of TE or TM radiation during capture of the output of desired TM or TE radiation respectively. This may consist of an electro-optic half wave plate placed in series with an analyser. A controller (35) is arranged to synchronise the source (31), the analyser system (37) and the photodiode array (34).

The interferometric sensor component (13) of the sensor device used in the following Examples is illustrated in Figure 6. The interferometric sensor component (13) is a laminate structure consisting of an absorbent sensing layer (1) separated from a reference waveguide (3) by a silicon dioxide spacer (2). A further silicon dioxide spacer (4) separates the silicon oxynitride reference waveguide (3) from a substrate (5) of silicon.

**Example 1: Immobilisation of pre-silanised oligonucleotides**

[(3-mercaptopropyl)-trimethoxysilane] was diluted to 5 mM stock solution in sodium acetate buffer (30mM, pH 4.3). 3$\mu$l of thiol-labelled oligo (thiol, 3nMol) were incubated with 3$\mu$l 5mM mercaptosilane solution and 54$\mu$l sodium acetate buffer for 2 hours at room temperature. Sensor components were immersed in 10% NaOH for 30 minutes, washed thoroughly with distilled water and dried for at least 15 minutes at 80°C.

In order to chemically attach the oligonucleotide strand to the sensor component surface, the silanised nucleic acids were pipetted on the sensor components, incubated for 15 minutes at room temperature and dried at 50°C for 5 minutes. The sensorcomponents were dipped in hot water (80°C; 30 s) to remove non-covalently bound oligonucleotides and dried at 50°C.

To investigate the unspecific binding of non-complementary oligonucleotide, 200$\mu$l of 1$\mu$M oligo 7A3H were injected into the sample loop. After complete coverage of the sensor component surface with test solution the flow was stopped for 20 minutes.

Prior to hybridisation of complementary oligonucleotide, non tightly bound non-complementary oligonucleotide was removed by washing the surface with 2xSSC until a stable baseline was reached. Subsequently, 200 $\mu$l complementary strand RP4T was injected and incubated for 20 minutes on the sensor surface. This was followed by a washing step with running buffer. 50 mM NaOH was applied to denature the double-strand on the surface and to make it available for a second hybridisation reaction.

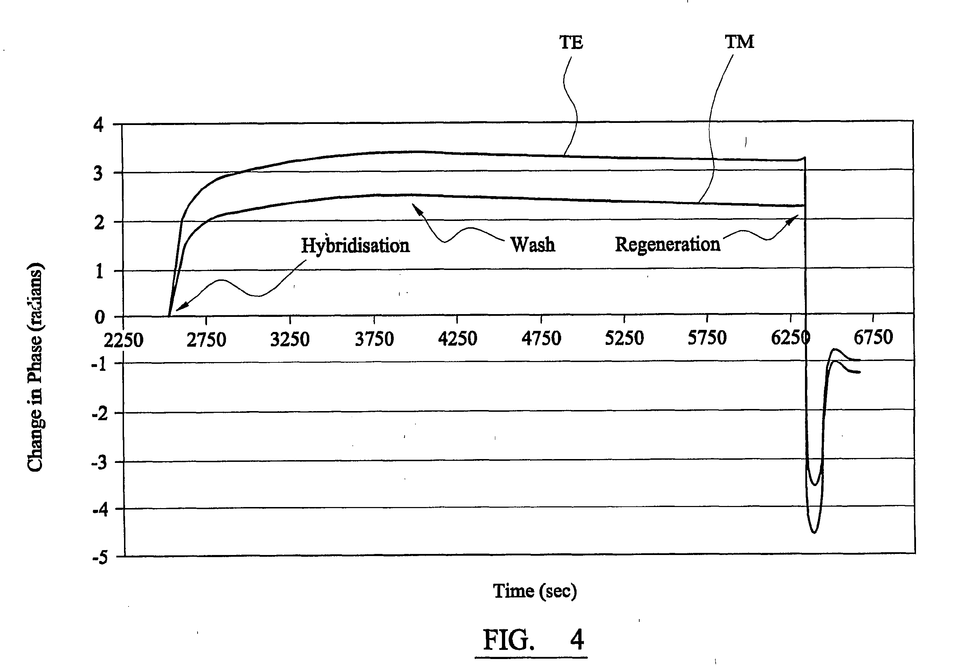

The onset of hybridisation is shown in figure 4 where the complimentary strand is added at 2500s. This binding is stable since it is not reversed when oligonucleotide free buffer solution is washed over the sensor component at 4000s.

Changing the pH or raising the temperature disrupts the hybridisation. As seen in Figure 4, sodium hydroxide solution is added at 6300s and flushed through thereby removing the hybridised complimentary strand and effectively regenerating the surface.

Figure 4 shows that the TE response is larger than the TM response. This implies a significant structural change in the surface morphology of the film (ie a reduction in thickness) as one would expect from the formation of a hybridised, double helical structure collapsing onto the surface of the sensor component. Furthermore, the response is of the opposite sense to that of non-specific molecular attachment in which the TM response would be larger than the TE response.

The effect of the decrease in thickness with the increase in mass associated with the specific binding event results in a substantial increase in the molecular density of the sensing layer. Non-specific molecular attachment by comparison results in an increase in both layer thickness and mass and result in substantially the same molecular density. Sensitivity of the sensing layer to the molecular density (as opposed to the mass) allows differentiation between specific and non-specific binding and greatly increases the signal to noise ratio and therefore the utility of the sensor device (probe). This is generally true for any binding event but is especially so in the case of DNA hybridisation where the creation of the double helix structure is one of the largest changes in molecular density observable. A more general example of specific and non-specific binding is described below.

**Example 2: Determination of specific and non-specific binding in a metabolic pathway**

Fatty acid biosynthesis in plants is carried out by a number of soluble enzymes with each protein molecule containing a single activity. However, there is increasing evidence that consecutive enzymes in the metabolic sequence interact *in vivo* so that chemical intermediates are passed between them in a process known as substrate channelling.

A detailed understanding of the mechanisms involved in fatty acid synthesis will enable these processes to be manipulated. Plants are already used as bioreactors for the synthesis of useful molecules and foodstuffs which are harvested from the plants. By either disrupting the synthetic pathway (or perhaps by substituting an alternative protein) the range and quantities of these plant products could be expanded to include useful compounds that are at present not made in specific plants or to improve the yield of those that are.

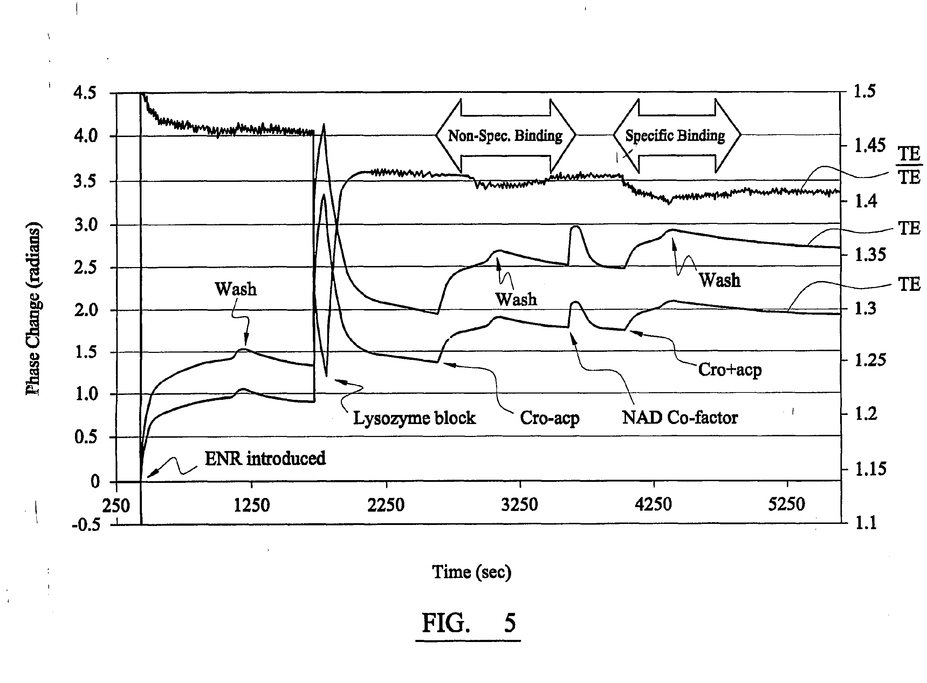

Figure 5 shows the immobilization of enolreductase (ENR - an enzyme involved in fatty acid biosynthesis) on a chemically activated sensor component at 400s. The surface of the sensor component is then blocked with lysozyme at 1700s. At 2600s, Cro-acp (the enzyme's substrate) is first introduced. This is not expected to bind without the cofactor NAD+ but seems to do so, demonstrating a non-specific event. At 3600s, NAD+ is added and some of this small molecule binds to the structure. When Cro-acp is added again, more binds to the surface.

With the benefit of TE and TM mode electromagnetic irradiation, the sequence of events occurring during this experiment can be qualitatively interpreted. ENR forms as a film on the surface, the lysozyme can be seen to sorb into the film and is mostly washed away again. The first Cro-acp does not change the molecular density of the film very much (the TE/TM ratio is constant) but addition of the second aliquot (after addition of the NAD+) causes a noticeable densification of the film (the TE/TM ratio reduces sharply). Non specific adhesion can therefore be distinguished from the specific binding generated by the introduction of the cofactor NAD+.

**Example 3: Analysis of slab waveguide structures using EM field theory**

Generally, the intrinsic refractive index and thickness can be resolved from the measurement of effective refractive index by the solution of Maxwell's equations for propagation of electromagnetic radiation through the combined sensing system and analyte.

*A - Three layer waveguide structure*

For a general three layer (slab) waveguide structure, it is possible to derive an eigenvalue equation for each polarisation. For TE modes:

$$\alpha_1 t = \tan^{-1}(\alpha_2/\alpha_1) + \tan^{-1}(\alpha_3/\alpha_1) + m\pi \quad (m = 0,1,2...)$$

where m is the mode order and

$$\alpha_1^2 = \left(n_1^2 k_0^2 - \beta_m^2\right) \;;\; \alpha_3^2 = \left(\beta_m^2 - n_3^2 k_0^2\right) \;;\; \alpha_2^2 = \left(\beta_m^2 - n_2^2 k_0^2\right)$$

and $\beta_m = n_{eff,m} k_0$.
(where the core layer is 1, substrate and cladding layers 2 and 3, thickness of the core layer t and the free space

wavenumber $k_0 = \dfrac{2\pi}{\lambda_0}$ ).

For TM mode the equation is:

$$\alpha_1 t = \tan^{-1}\left(\frac{n_1^2}{n_2^2}\alpha_2/\alpha_1\right) + \tan^{-1}\left(\frac{n_1^2}{n_3^2}\alpha_3/\alpha_1\right) + m\pi$$

Generally, these equations are solved for the effective refractive index values by numerical techniques given a known set of refractive index and thickness parameters. Alternatively if the absolute effective index value is known, it is possible to solve for one variable of the system for each equation given that the other three are known.

In the sensor, the starting effective refractive index value ($n_{eff}(0)$) is known since the intrinsic refractive index and thickness parameters of the system are known. This would be obtained by numerical solving of either of the above for the relevant polarisation. The sensor would then record an effective refractive index difference ($\Delta n_{eff}$) upon a new cladding layer being introduced (eg a binding event). An absolute effective index $n_{eff}(1)=n_{eff}(0)+\Delta n_{eff}$ could be put in to either of the two equations above to solve for the refractive index of the cladding layer.

*B - Four and more layer waveguide structure*

To analyse more complicated structures where the number of parameters can be arbitrarily large requires matrix methods to reduce the complexity. The equation to solve becomes:

$$ j(\alpha_s m_{11} + \alpha_c m_{22}) = m_{21} - \alpha_s \alpha_c m_{12} $$

where $\alpha_s$ and $\alpha_c$ refer to the terms for substrate and cladding, the two outer bounding layers, within which the field has a decaying exponential form. They are written as:

$$ \alpha_s^2 = \left(\beta_m^2 - n_s^2 k_0^2\right) \quad \text{and} \quad \alpha_c^2 = \left(\beta_m^2 - n_c^2 k_0^2\right) $$

The $m_{ij}$ terms are elements of a 2 × 2 matrix which is the resultant matrix formed by multiplication of the individual characteristic matrix of each layer. Thus for each layer within the stack (not the bounding layers) we have for TE mode;

$$ M_i = \begin{vmatrix} \cos(\alpha_i t_i) & j/\alpha_i \sin(\alpha_i t_i) \\ j\alpha_i \sin(\alpha_i t_i) & \cos(\alpha_i t_i) \end{vmatrix} $$

where $t_i$ is the thickness of each layer, and the index of each layer is;

$$ \alpha_i^2 = \left(n_i^2 k_0^2 - \beta_m^2\right) $$

The characteristic matrix (M) for the whole stack is obtained through multiplication as follows;

$$ M = \begin{vmatrix} m_{11} & m_{12} \\ m_{21} & m_{22} \end{vmatrix} = M_1 \cdot M_2 \cdot M_3 \cdot ..... M_n $$

For TM modes we modify the terms in the characteristic matrix for each layer
The equation must be initially solved numerically using a known structure and calculating the TE effective index. The sensor would then provide a new TE effective index after layer deposition. This would be fed into the multilayer equation and solved for one parameter (either thickness or refractive index) of the film, setting the other parameter as a range variable. The analogous process could be carried out using the corresponding TM result. The unique combination of intrinsic refractive index and thickness would be found by correlating the TE and TM results as shown in figure 7. The crossing point is the unique solution for intrinsic refractive index and thickness for the combined sensor and analyte system.

From the intrinsic refractive index one can determine density and the absolute thickness is directly related to volume from which the absolute mass follows (density x volume = mass). Furthermore it is generally known that proteins may be discriminated by mass which can be determined from the calculated density if the thickness is determined as per the previous example.

Although intrinsic refractive index is not directly related to density, if we make certain assumptions regarding the system under investigation (eg water has zero protein density at a refractive index of 1.33 and a known protein (eg streptavidin) has a refractive index of 1.45 for a known density) then generally one can attribute a change in refractive index to a change in percentage protein density:

```
Density(unknown protein) = Density(protein standard)*(RI(unknown protein)-RI(water))
                           --------------------------------------------
                                  (RI(protein standard)-RI(water))
```

Absolute molecular density is a fundamental characteristic of any molecule and its determination and discrimination can therefore be used as a differentiating molecular signature.

## Claims

1. A method for determining a mass changing event in a material of interest in a localised environment, said method comprising:

   (A) providing a sensor device having a sensor component (13) capable of exhibiting a measurable response to a change in the localised environment caused by the mass changing event in the material of interest therein, wherein the sensor component (13) is a waveguide structure including: either (a) one or more sensing layers capable of inducing in a secondary waveguide a measurable response to a change in the localised environment caused by the mass changing event and an inactive secondary waveguide in which the sensing layer is incapable of inducing a measurable response to a change in the localised environment caused by the mass changing event or (b) a sensing waveguide (1) capable of exhibiting a measurable response to a change in the localised environment caused by the mass changing event and an inactive waveguide (3) substantially incapable of exhibiting a measurable response to a change in the localised environment caused by the mass changing event.
   (B) introducing the material of interest into the localised environment;
   (C) inducing the mass changing event in the material of interest;
   (D) generating an output from the sensor component over a temporal range, wherein step (D) comprises:

      (D1) irradiating the sensor component with electromagnetic radiation in TE mode to produce a first pattern of interference fringes;
      (D2) irradiating the sensor component with electromagnetic radiation in TM mode to produce a second pattern of interference fringes;

   (E) measuring the response of a characteristic of the output over the temporal range, wherein step (E) comprises:

      (E1) measuring movements in the first pattern of interference fringes;
      (E2) measuring movements in the second pattern of interference fringes;
      (E3) calculating the phase shift of the sensor component in TM mode from the movements in the first pattern of interference fringes
      (E4) calculating the phase shift of the sensor component in TE mode from the movements in the second pattern of interference fringes;
      (E5) calculating the phase shift of the sensor component in TM mode relative to the phase shift of the sensor component in TE mode; and

(F) relating the phase shift of the sensor component in TM mode relative to the phase shift of the sensor component in TE mode to the mass changing event.

2. A method as claimed in claim 1 wherein the mass changing event is a chemical mass changing event.

3. A method as claimed in claim 2 wherein the mass changing event is a specific molecular (or atomic) interaction.

4. A method as claimed in claim 3 wherein the mass changing event is a specific associative or dissociative molecular interaction.

5. A method as claimed in any of claims 1 to 4 wherein the mass changing event is a binding event.

6. A method as claimed in any of claims 1 to 5 wherein the mass changing event is a specific binding event.

7. A method as claimed in any of claims 1 to 5 wherein the binding event is a bond making or bond breaking event.

8. A method as claimed in any of claims 1 to 5 wherein the binding event is an associative event or a dissociative event.

9. A method as claimed in claim 8 wherein the binding event is formation of a molecular composition or decomposition of a molecular composition.

10. A method as claimed in any of claims 5 to 9 wherein the material of interest is a biological molecule.

11. A method as claimed in claim 10 wherein the biological molecule is an antigen and the binding event is formation of an antibody/antigen specific binding pair.

12. A method as claimed in claim 1 wherein the mass changing event is a conformational change of the material of interest.

13. A method as claimed in claim 12 wherein the conformational change is a molecular rearrangement.

14. A method as claimed in claim 1 wherein the material of interest is a nucleotide and the mass changing event is effective nucleotide complementation.

15. A method as claimed in claim 1 or 2 wherein the mass changing event is a physical mass changing event.

16. A method as claimed in claim 15 wherein the mass changing event is aggregation.

17. A method as claimed in any preceding claim wherein step (C) comprises: imposing a condition such as to induce the mass changing event.

18. A method as claimed in claim 17 wherein the condition is selected from the group consisting of a chosen temperature, pressure, acidity, solvent and humidity.

19. A method as claimed in any preceding claim wherein the sensor device is an interferometric sensor device.

20. A method as claimed in claim 1 wherein each of the sensing waveguide (1) or secondary waveguide of the sensor component is a planar waveguide.

21. A method as claimed in any preceding claim wherein the mass changing event contributes to a change in the effective refractive index of the sensor component (13).

22. A method as claimed in claim 1 wherein the phase shift of the sensor component in TM mode relative to the phase shift of the sensor component (13) in TE mode is a ratio of the phase shift of the sensor component in TM mode to the phase shift of the sensor component in TE mode.

23. A method as claimed in any of claims 1 to 22 further comprising:

(G1) relating the movements in the first pattern of interference fringes and second pattern of interference fringes to a change in the intrinsic refractive index and/or the volume; and

(G2) calculating the change in the molecular density; and

(G3) optionally calculating the change in mass.

**24.** A method as claimed in claim 23 wherein step (G1) comprises:

(G1) relating the movements in the first pattern of interference fringes and second pattern of interference fringes to a change in the intrinsic refractive index and/or the thickness of the sensing layer or sensing waveguide.

**25.** A method as claimed in any of claims 19 to 24 wherein the sensor device further comprises: means for intimately exposing at least a part of the (or each) sensing layer or the sensing waveguide (1) of the sensor component (13), said means defining the localised environment having a volume of 50 microlitres or less.

**26.** A method as claimed in any preceding claim wherein step (D) comprises: generating an output from the sensor component (13) on at least two occasions over a temporal range.

**27.** A method as claimed in claim 26 wherein step (D) comprises: generating an output from the sensor component (13) continuously over a temporal range.


**Patentansprüche**

**1.** Verfahren zum Bestimmen eines Masseänderungsereignisses in einem Material unter Beobachtung in einer lokalisierten Umgebung, wobei das Verfahren umfasst:

(A) Bereitstellen eines Sensorgeräts mit einer Sensorkomponente (13), die eine messbare Antwort auf eine Änderung in der lokalisierten Umgebung, die durch das Masseänderungsereignis in dem Material unter Beobachtung darin bewirkt wird, aufweisen kann, wobei die Sensorkomponente (13) eine Wellenleiterstruktur ist, die beinhaltet: entweder (a) eine oder mehrere Sensorschichten, die in einem sekundären Wellenleiter eine messbare Antwort auf eine Änderung in der lokalisierten Umgebung, die durch das Masseänderungsereignis bewirkt wird, induzieren können und einen inaktiven sekundären Wellenleiter, in welchem die Sensorschicht keine messbare Antwort auf eine Änderung in der lokalisierten Umgebung, die durch das Masseänderungsereignis bewirkt wird, induzieren kann, oder (b) einen Sensorwellenleiter (1), der eine messbare Antwort auf eine Änderung in der lokalisierten Umgebung, die durch das Masseänderungsereignis bewirkt wird, aufweisen kann und einen inaktiven Wellenleiter (3), der im Wesentlichen keine messbare Antwort auf eine Änderung in der lokalisierten Umgebung, die durch das Masseänderungsereignis bewirkt wird, aufweisen kann,

(B) Einbringen des Materials unter Beobachtung in die lokalisierte Umgebung;

(C) Induzieren des Masseänderungsereignisses in dem Material unter Beobachtung;

(D) Erzeugen einer Ausgabe der Sensorkomponente über einem Zeitbereich, wobei Schritt (D) umfasst:

(D1) Bestrahlen der Sensorkomponente mit elektromagnetischer Strahlung in einer TE-Mode, um ein erstes Muster von Interferenzlinien zu produzieren;

(D2) Bestrahlen der Sensorkomponente mit elektromagnetischer Strahlung in einer TM-Mode, um ein zweites Muster von Interferenzlinien zu produzieren;

(E) Messen der Antwort einer Charakteristik der Ausgabe über dem Zeitbereich, wobei Schritt (E) umfasst:

(E1) Messen von Bewegungen in dem ersten Muster von Interferenzlinien;

(E2) Messen von Bewegungen in dem zweiten Muster von Interferenzlinien;

(E3) Berechnen der Phasenverschiebung der Sensorkomponente in der TM-Mode aus den Bewegungen in dem ersten Muster von Interferenzlinien;

(E4) Berechnen der Phasenverschiebung der Sensorkomponente in der TE-Mode aus den Bewegungen in dem zweiten Muster von Interferenzlinien;

(E5) Berechnen der Phasenverschiebung der Sensorkomponente in der TM-Mode relativ zu der Phasenverschiebung der Sensorkomponente in der TE-Mode; und

(F) In-Bezug-Setzen der Phasenverschiebung der Sensorkomponente in der TM-Mode relativ zu der Phasenverschiebung der Sensorkomponente in der TE-Mode zu dem Masseänderungsereignis.

2. Verfahren wie in Anspruch 1 beansprucht, wobei das Masseänderungsereignis ein chemisches Masseänderungsereignis ist.

3. Verfahren wie in Anspruch 2 beansprucht, wobei das Masseänderungsereignis eine spezifische molekulare (oder atomare) Wechselwirkung ist.

4. Verfahren wie in Anspruch 3 beansprucht, wobei das Masseänderungsereignis eine spezifische assoziative oder dissoziative Molekularwechselwirkung ist.

5. Verfahren wie in einem der Ansprüche 1 bis 4 beansprucht, wobei das Masseänderungsereignis ein Bindungsereignis ist.

6. Verfahren wie in einem der Ansprüche 1 bis 5 beansprucht, wobei das Masseänderungsereignis ein spezifisches Bindungsereignis ist.

7. Verfahren wie in einem der Ansprüche 1 bis 5 beansprucht, wobei das Bindungsereignis ein bindungsausbildendes oder bindungsaufbrechendes Ereignis ist.

8. Verfahren wie in einem der Ansprüche 1 bis 5 beansprucht, wobei das Bindungsereignis ein assoziatives Ereignis oder ein dissoziatives Ereignis ist.

9. Verfahren wie in Anspruch 8 beansprucht, wobei das Bindungsereignis Ausbildung einer molekularen Zusammensetzung oder Auflösung einer molekularen Zusammensetzung ist.

10. Verfahren wie in einem der Ansprüche 5 bis 9 beansprucht, wobei das Material unter Beobachtung ein biologisches Molekül ist.

11. Verfahren wie in Anspruch 10 beansprucht, wobei das biologische Molekül ein Antigen ist und das Bindungsereignis Ausbildung eines Antikörper-/Antigen-spezifischen Bindungspaares ist.

12. Verfahren wie in Anspruch 1 beansprucht, wobei das Masseänderungsereignis eine konformative Änderung des Materials unter Beobachtung ist.

13. Verfahren wie in Anspruch 12 beansprucht, wobei die konformative Änderung eine molekulare Neuanordnung ist.

14. Verfahren wie in Anspruch 1 beansprucht, wobei das Material unter Beobachtung ein Nukleotid ist und das Masseänderungsereignis eine effektive Nukleotidvervollständigung ist.

15. Verfahren wie in Anspruch 1 oder 2 beansprucht, wobei das Masseänderungsereignis ein physikalisches Masseänderungsereignis ist.

16. Verfahren wie in Anspruch 15 beansprucht, wobei das Masseänderungsereignis Aggregation ist.

17. Verfahren wie in einem der voranstehenden Ansprüche beansprucht, wobei Schritt (C) umfasst: Bewirken einer Bedingung, sodass das Masseänderungsereignis induziert wird.

18. Verfahren wie in Anspruch 17 beansprucht, wobei die Bedingung aus der Gruppe ausgewählt wird, die besteht aus einer ausgewählten Temperatur, Druck, Säuregrad, Lösungsmittel und Feuchtigkeit.

19. Verfahren wie in einem der voranstehenden Ansprüche beansprucht, wobei die Sensorvorrichtung eine interferometrische Sensorvorrichtung ist.

20. Verfahren wie in Anspruch 1 beansprucht, wobei entweder der Sensorwellenleiter (1) oder der sekundäre Wellenleiter der Sensorkomponente ein planarer Wellenleiter ist.

21. Verfahren wie in einem der voranstehenden Ansprüche beansprucht, wobei das Masseänderungsereignis zu einer Änderung des effektiven Brechungsindex der Sensorkomponente (13) beiträgt.

22. Verfahren wie in Anspruch 1 beansprucht, wobei die Phasenverschiebung der Sensorkomponente in der TM-Mode relativ zu der Phasenverschiebung der Sensorkomponente (13) in der TE-Mode ein Bruchteil der Phasenverschiebung der Sensorkomponente in der TM-Mode zu der Phasenverschiebung der Sensorkomponente in der TE-Mode ist.

23. Verfahren wie in einem der Ansprüche 1 bis 22 beansprucht, weiterhin umfassend:

(G1) In-Bezug-Setzen der Bewegungen des ersten Musters von Interferenzlinien und des zweiten Musters von Interferenzlinien zu einer Änderung des intrinsischen Brechungsindexes und/oder des Volumens; und
(G2) Berechnen der Änderung der molekularen Dichte; und
(G3) optional Berechnen der Änderung der Masse.

24. Verfahren wie in Anspruch 23 beansprucht, wobei Schritt (G1) umfasst:

(G1) In-Bezug-Setzen der Bewegungen in dem ersten Muster von Interferenzlinien und dem zweiten Muster von Interferenzlinien zu einer Änderung des intrinsischen Brechungsindexes und/oder der Dicke der Sensorschicht oder des Sensorwellenleiters.

25. Verfahren wie in einem der Ansprüche 19 bis 24 beansprucht, wobei die Sensorvorrichtung weiterhin umfasst: Mittel zum genauen Belichten von mindestens einem Teil der (oder von einem der beiden) Sensorschicht oder des Sensorwellenleiters (1) der Sensorkomponente (13), wobei die Mittel die lokalisierte Umgebung mit einem Volumen von 50 ml oder weniger definieren.

26. Verfahren wie in einem der voranstehenden Ansprüche beansprucht, wobei Schritt (D) umfasst: Erzeugen einer Ausgabe aus der Sensorkomponente (13) bei zumindest zwei Ereignispunkten über einem Zeitbereich.

27. Verfahren wie in Anspruch 26 beansprucht, wobei Schritt (D) umfasst: Erzeugen einer Ausgabe der Sensorkomponente (13) kontinuierlich über einen Zeitbereich.

## Revendications

1. Procédé destiné à déterminer un événement de changement de masse d'un matériau d'intérêt dans un environnement localisé, ledit procédé comprenant :

(A) la fourniture d'un dispositif de détection présentant un composant de détection (13) capable de montrer une réponse mesurable à un changement dans l'environnement localisé, provoqué par l'événement de changement de masse à l'intérieur du matériau d'intérêt, dans lequel le composant de détection (13) est une structure de guide d'ondes comprenant: soit (a) une ou plusieurs couches de détection capables d'induire dans un guide d'ondes secondaire une réponse mesurable à un changement dans l'environnement localisé, provoqué par l'événement de changement de masse et un guide d'ondes secondaire inactif dans lequel la couche de détection est incapable d'induire une réponse mesurable à un changement dans l'environnement localisé, provoqué par l'événement de changement de masse, soit (b) un guide d'ondes de détection (1) capable de montrer une réponse mesurable à un changement dans l'environnement localisé, provoqué par l'événement de changement de masse et un guide d'ondes inactif (3) sensiblement incapable de montrer une réponse mesurable à un changement dans l'environnement localisé, provoqué par l'événement de changement de masse ;
(B) l'introduction du matériau d'intérêt dans l'environnement localisé ;
(C) la provocation de l'événement de changement de masse dans le matériau d'intérêt ;
(D) le fait de générer une sortie en provenance du composant de détection sur une plage temporelle, dans lequel l'étape (D) comprend :

(D1) l'irradiation du composant de détection avec un rayonnement électromagnétique dans un mode TE pour produire un premier motif de franges d'interférence ;
(D2) l'irradiation du composant de détection avec un rayonnement électromagnétique dans un mode TM pour produire un second motif de franges d'interférence ;

(E) la mesure de la réponse d'une caractéristique de la sortie sur la plage temporelle, dans lequel l'étape (E) comprend :

(E1) la mesure des mouvements dans le premier motif de franges d'interférence ;

(E2) la mesure des mouvements dans le second motif de franges d'interférence ;

(E3) le calcul du déphasage du composant de détection dans le mode TM à partir des mouvements dans le premier motif de franges d'interférence ;

(E4) le calcul du déphasage du composant de détection dans le mode TE à partir des mouvements dans le second motif de franges d'interférence ;

(E5) le calcul du déphasage du composant de détection en mode TM par rapport au déphasage du composant de détection en mode TE ; et

(F) mise en relation du déphasage du composant de détection en mode TM par rapport au déphasage du composant de détection en mode TE, avec l'événement de changement de masse.

2. Procédé selon la revendication 1, dans lequel l'événement de changement de masse est un événement de changement de masse chimique.

3. Procédé selon la revendication 2, dans lequel l'événement de changement de masse est une interaction moléculaire (ou atomique) spécifique.

4. Procédé selon la revendication 3, dans lequel l'événement de changement de masse est une interaction moléculaire associative ou dissociative spécifique.

5. Procédé selon l'une quelconque des revendications 1 à 4, dans lequel l'événement de changement de masse est un événement de liaison.

6. Procédé selon l'une quelconque des revendications 1 à 5, dans lequel l'événement de changement de masse est un événement de liaison spécifique.

7. Procédé selon l'une quelconque des revendications 1 à 5, dans lequel l'événement de liaison est un événement d'établissement de liaison ou de rupture de liaison.

8. Procédé selon l'une quelconque des revendications 1 à 5, dans lequel l'événement de liaison est un événement associatif ou un événement dissociatif.

9. Procédé selon la revendication 8, dans lequel l'événement de liaison est la formation d'une composition moléculaire ou la décomposition d'une composition moléculaire.

10. Procédé selon l'une quelconque des revendications 5 à 9, dans lequel le matériau d'intérêt est une molécule biologique.

11. Procédé selon la revendication 10, dans lequel la molécule biologique est un antigène et l'événement de liaison est la formation d'une paire de liaison spécifique anticorps/antigène.

12. Procédé selon la revendication 1, dans lequel l'événement de changement de masse est un changement de conformation du matériau d'intérêt.

13. Procédé selon la revendication 12, dans lequel le changement de conformation est un remaniement moléculaire.

14. Procédé selon la revendication 1, dans lequel le matériau d'intérêt est un nucléotide et l'événement de changement de masse est une complémentation de nucléotide effective.

15. Procédé selon la revendication 1 ou 2, dans lequel l'événement de changement de masse est un événement de changement de masse physique.

16. Procédé selon la revendication 15, dans lequel l'événement de changement de masse est une agrégation.

17. Procédé selon l'une quelconque des revendications précédentes, dans lequel l'étape (C) comprend : le fait d'imposer une condition qui provoque l'événement de changement de masse.

**18.** Procédé selon la revendication 17, dans lequel la condition est sélectionnée dans le groupe constitué par une température, une pression, une acidité, un solvant et une humidité choisis.

**19.** Procédé selon l'une quelconque des revendications précédentes, dans lequel le dispositif de détection est un dispositif de détection interférométrique.

**20.** Procédé selon la revendication 1, dans lequel chacun parmi le guide d'ondes de détection (1) ou le guide d'ondes secondaire du composant de détection est un guide d'ondes planaire.

**21.** Procédé selon l'une quelconque des revendications précédentes, dans lequel l'événement de changement de masse contribue à un changement de l'indice de réfraction effectif du composant de détection (13).

**22.** Procédé selon la revendication 1, dans lequel le déphasage du composant de détection en mode TM par rapport au déphasage du composant de détection (13) en mode TE est le rapport du déphasage du composant de détection en mode TM par rapport au déphasage du composant de détection en mode TE.

**23.** Procédé selon l'une quelconque des revendications 1 à 22 comprenant en outre :

(G1) la mise en relation des mouvements dans le premier motif de franges d'interférence et le second motif de franges d'interférence, avec un changement de l'indice de réfraction intrinsèque et/ou du volume ; et
(G2) le calcul du changement de la densité moléculaire ; et
(G3) le calcul éventuel du changement de masse.

**24.** Procédé selon la revendication 23, dans lequel l'étape (G1) comprend :

(G1) la mise en relation des mouvements dans le premier motif de franges d'interférence et le second motif de franges d'interférence, avec un changement de l'indice de réfraction intrinsèque et/ou de l'épaisseur de la couche de détection ou du guide d'ondes de détection.

**25.** Procédé selon l'une quelconque des revendications 19 à 24, dans lequel le dispositif de détection comprend en outre : des moyens pour exposer en association étroite au moins une partie de la (ou de chaque) couche de détection ou le guide d'ondes de détection (1) du composant de détection (13), lesdits moyens définissant l'environnement localisé présentant un volume inférieur ou égal à 50 microlitres.

**26.** Procédé selon l'une quelconque des revendications précédentes, dans lequel l'étape (D) comprend : le fait de générer une sortie en provenance du composant de détection (13) lors de deux occasions au moins sur une plage temporelle.

**27.** Procédé selon la revendication 26, dans lequel l'étape (D) comprend : le fait de générer une sortie en provenance du composant de détection (13) de manière continue sur une plage temporelle.

FIG. 1

FIG. 2

**FIG. 3**

**FIG. 7**

FIG. 4

FIG. 5

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5120131 A
- US 6127183 A
- US 5501986 A

- WO 9822807 A
- WO 0136945 A
- GB 0207167 A

**Non-patent literature cited in the description**

- **LU et al.** *J. Chem. Soc. Far Trans.,* 1994, vol. 8, 9951018